# EUROPEAN PATENT APPLICATION

(11) **EP 2 735 311 A1**
(43) Date of publication of application: **28.05.2014**
(21) Application number: 12802772.9
(22) Date of filing: 25.06.2012
(51) Int. Cl.: A61K 36/38, C07D 311/08, A61P 31/04

(54) **PLANT EXTRACT OBTAINED FROM KIELMEYERA AUREOVINOSA WHICH HAS ANTIBIOTIC ACTIVITY, ISOLATED CHEMICAL COMPOUND, COMPOSITIONS COMPRISING SAME, USES THEREOF AND METHODS FOR PREVENTING AND TREATING BACTERIAL INFECTIONS**

(30) Priority: 22.06.2011 US 201161500077 P
(71) Applicant: Extracta Moléculas Naturais S/A, Rio de Janeiro - RJ 21.941-904 (BR)
(72) Inventor: PAES DE CARVALHO, Antônio, CEP : 22.610-010 Rio de Janeiro (RJ) (BR); PADULA DE MIRANDA, Otavio, CEP : 25.610-010 Petrópolis (RJ) (BR); DE SANTANA JULIÃO, Lisieux, CEP: 26.600-000 Paracambi (RJ) (BR); MARIE ACCIOLY MACHADO DE OLIVEIRA, Giselle, CEP : 21.920-630 Rio de Janeiro (RJ) (BR); GOMES, Mário, CEP : 22.460-030 Rio de Janeiro (RJ) (BR)
(74) Representative: Potter Clarkson LLP
(86) International application number: PCT/BR2012/000190
(87) International publication number: WO 2012/174622

(57) **Abstract**

The present invention refers primarily to a plant extract obtained from *Kielmeyera aureovinosa* which has accentuated antibiotic activity. In a second modality, the invention describes a new chemical compound isolated from the same, as well as variations thereof. In other terms, the present invention provides compositions comprising a phytotherapic obtained from said plant extract, as well as veterinary pharmaceutical compositions comprising isolated compounds and variations of the same.

Additionally, the present invention provides uses of the plant extract, chemical compounds and compositions comprising the same for the preparation of medicines for the prevention or treatment of bacterial infections, as well as methods of prevention or treatment of the same.

## Description

The present invention refers to plant extracts obtained from plant species *Kielmeyera aureovinosa,* Family *Clusiaceae,* native and collected in Brazil, which has marked antibiotic activity.

The present invention also refers to the chemical compound 5-hydroxy-8,8-dimethyl-6-(iso-propyl)-4-aryl-4H-cyclohexen-(2,3-h]chromen-2-one (amarilin), isolated from the plant extract of *Kielmeyera aureovinosa,* which compound also features high antibiotic activity, as well as variations of the same.

The present invention also relates to phytotherapic (herbal) compositions obtained from said plant extract of *Kielmeyera aureovinosa.*

The present invention also provides pharmaceutical or veterinary compositions comprising the active compound amarilin.

The present invention also provides for the use of these compositions in the prevention or treatment of bacterial infections.

Finally, the present invention relates to methods to prevent or treat bacterial infections.

### BACKGROUND OF THE INVENTION

### On phytotherapic (herbal) medicines

Currently, it is estimated that approximately 25% of the medicines produced and distributed internationally include substances of plant origin as active compounds (Bourgaud, F.; Gravot, A.; Milesi, S.; Gontier, E. (2001), Production of plant secondary metabolites: a historical perspective. Plant science, v.161, p. 839-851). Of 877 new small chemical molecules introduced as drugs in the period from 1981 to 2002, 61% had their origin or were inspired by natural compounds (NEWMAN, D. J., CRAGG, G. M., SNADER K. M. 2003. Natural Products the Sources of New Drugs over the Period 1981-2002. J. Nat. Prod., 66, 1022-1037), including natural products isolated directly (6%), their semi-synthetic derivatives (27%), synthetic compounds with pharmacophores derived from natural products (5 %) and synthetic compounds modeled from natural molecules, that is, mimicking natural products (23%).

The use of medicinal herbs and products derived from the same is well diffused in traditional medicine, which is based on the experience documented by man, in therapy and prophylaxis of various diseases. Such products have gained increasing popularity in the last decade even in developed countries, where they are used by approximately 20% of their populations. Phytotherapeutic Medicines are complex mixtures of organic substances that can come from any part of a plant, raw or processed, including leaves, stems, flowers, roots and seeds.

In developing countries, approximately 80% of the population depends on traditional forms of therapy, including medicines derived from plants, for health maintenance and for treatment of diseases (B. VAN BREEMEN, Richard, FONG, Harry H. S. and FARNSWORTH, Norman R., The Role of Quality Assurance and Standardization in the Safety of Botanical Dietary Supplements. Chem Res Toxicol 2007 April; 20 (4): 577- 582). This reality is widespread in countries of Latin America and Africa, for example.

### On Flavanoids

Among the group of important natural chemical substances found in plants are the flavonois. Flavonoids represent one of the largest and most widely distributed chemical groups among botanical families, covering several classes of chemical molecules having in common a basic structure C₆-C₃-C₆, and are classified according to the degree of oxidation of their piranic central nucleus, which can also be linear (as in chalcones) or fused to a furanic ring (as in aurones).

A diet based on vegetables is rich in flavonoids. It is believed that these are extremely important molecules for human health because they act as natural antioxidants, thus providing protection against cardiovascular diseases and certain types of cancer (DEWICK, P. M. 1997. Medicinal Natural Products - The Biossynthetic Approach, John Wiley & Sons Ed., Chichester, England).

One of the classes of substances of natural origin belonging to the flavonoid group are neoflavonoids, which are molecules composed of 15 carbon atoms grouped according to their molecular structure and origin. Among the types of compounds belonging to this class are the 4-arilcoumarines and the dalbergiones.

It is known that certain neoflavonoids present antimicrobial anti-protozoan, cytotoxic and quimiopreventive activity, and show reverse transcriptase inhibition specific for human immunodeficiency virus type 1 (HIV-1) (PATIL, A. D. , FREYER's RETRACTOR, A. J. , EGGLESTON, D. S. , HALTIWANGER, R. C. , BEAN, M. F. , TAYLOR, P. B. , , CARANFA, M. J., BREEN, A. L. , , BARTUS, H. R. , JOHNSONR. K. , HERTZBERG, R. P. & WESTLEY, J. W. 1993. The inophyllums, novel inhibitors of HIV-1 reverse transcriptase isolated from the Malaysian tree, Calophyllum inophyllum Linn. (Journal of Medicinal Chemistry, 36, 4131-4137; ITOIGAWA, M. , ITO, C. , TAN, T. H. , KUCHIDE, M. , TOKUDA, H. , NISHINO, H. & FURUKAWA, H. 2001); Cancer chemopreventive agents, 4-phenylcoumarins from Calophyllum inophyllum. Cancer Letters, 169, 15- 19; B. M. W. OUAHOUO, A. G. B. AZEBAZE, M. MEYER, B. BODO, Z. T. FOMUM and A. E. NKENGFACK, Antimicrobial and Cytotoxic coumarins from Mammea africana. Annals of Tropical Medicine & Parasitology, Vol. 98, No. 7, 733-739 (2004)).

As previously mentioned, the flavonoids are widely distributed among the botanical families, among which we can highlight the family *Clusiaceae,* to which belongs the genus Kielmeyera. This Family presents about 1,000 species distributed in 30 genera found in the pantropical zone. In Brazil, there are 18 genera and about 150 species (SOUZA V., LORENZI H. 2005. Systematic Botany: illustrated guide to identification of families of angiosperms of Brazilian flora. Sao Paulo: Instituto Plantarum, p . 348), the genus Kielmeyera being endemic in South America (BENNETT, G. J. & LEE, H.-H.(1989), Xanthones from Guttiferae, Phytochemistry, 28, 967- 998). Studies with species of this genus have verified the occurrence of some classes of chemical substances, among them the 4-alkyl-coumarines and 4-aryl-coumarines (neoflavonoids) and the xanthones.

### On infections caused by multiresistant microorganisms

The nosocomial infections are considered as the main factor associated with the morbidity and mortality in nosocomial environments, in almost all Continents. In this aspect, the bacterial species *Staphylococcus aureus* has been considered the main etiologic agent associated with these infections, which can result in conditions of high morbidity such as infectious endocarditis and septicemias (SADER, H. S. ; WALES, A. C. ; PFALLER, M. A. ; ZOCCOLI, C. ; BARTH, & JONES, R. N. Pathogen frequency and resistance patterns in Brazilian hospitals. Summary of results from three years of the SENTRY antimicrobial surveillance program. Braz. J. Infect. Dis., 5, 200-214, 1999; ENRIGHT, M. C. 2003, Curr Opin Pharmacol. The evolution of a resistant pathogen - the case of MRSA. 3: 474-479; FOWLER, T. ; WANNÂ, E. R. ; JOH, D. ; JOHANSSON, S. ; Foster, T. J. & HOOK, M. Cellular invasion by Staphylococcus aureus involves a fibronectin bridge between the bacterial fibronectin-binding MSCRAMMS and host cell beta 1 integrins. Eur. J. Cell Biol., 79, 672-679, 2000; DIEKEMA DJ, PFALLER MA, SCHMITZ FJ, SMAYEVSKY J, BELL J, JONES RN, BEACH M; SENTRY Participants Group. Survey of infections due to Staphylococcus species: frequency of occurrence and antimicrobial susceptibility of isolates collected in the United States, Canada, Latin America, Europe, and the Western Pacific region for the SENTRY Antimicrobial Surveillance Program, 1997-1999. Clin Infect Dis. 32 Suppl 2:S114-32, 2001).

Hosptital outbreaks by multiresistant microorganisms (MCR) have been described postoperative associated with invasive procedures, as well with inadequate sanitization of instrumentation and/or surgical prostheses. The contamination leads to cutaneous infections in surgical wounds, cellulitis, abscesses with variable depth in different anatomical sites, post-aspiration pneumonia, keratitis, otitis media and mastoiditis, bacteremia, osteomyelitis, lymphadenitis, endocarditis related to cardiac surgeries and infections of central nervous system, such as meningitis.

The strains of *S. aureus* have a wide adaptive capacity, possessing various virulence factors, which have been associated with its high invasive capacity and pathogenic activity. Resistant variants of this species are widely disseminated in nosocomial environments, called "Methicillin-resistant Staphylococcus aureus" or MRSA. These strains generally present high levels of resistance to most classes of antimicrobial agents available for commercial and hospital use, what results in choice of empirical and inappropriate therapy, delay in the prescription of antimicrobials effective, as well as in use of less efficient, more toxic and more expensive drugs. All these factors contribute to hinder the epidemiological control and treatment of infections associated with this bacterial agent.

The methicillin resistance on the part of a microorganism is an indicator of resistance to all betalactam antibiotics, including cephalosporins and carbapenems. Resistant organisms exhibit often cross-resistance to aminoglycosides, macrolides, lincosamides, tetracyclines, trimethoprim and sulfonamides (RIBEIRO, Isabel and CASTANHEIRA, Rui. Treatment and prevention of infections and colonization by Staphylococcus aureus. In Rev Port Pneumol IX (5): 395-409. Porto, 2003).

The clinical samples of MRSA remained, for a long time, susceptible to glycopeptides (NNISS, 2001). However, since 1996, cases of intermediate resistance to vancomycin in S. aureus strains have been reported (HIRAMATSU, K.; HANAKI, H.; YNE, T.; YABUTA, K.; OGURI, T. & TENOVER, F. C. Methicillin-resistant Staphylococcus aureus clinical strain with reduced vancomycin susceptibility. J. Antimicrob. Chemother., 40, 135-136, 1997; SMITH, T. L. ; PEARSON, M. L. ; WILCOX, K. R. ; CRUZ, C. ; LANCASTER, M. V. ; SWANEY SM, AOKI H, GANOZA MC. The oxazolidinone linezolid inhibits initiation of protein synthesis in bacteria. Antimicrb Agents Chemother 1998 42:3251-3255) and more recently, sporadic cases in the United States and in Japan showed full resistance to this antibiotic (Centers of Diseases Control and Hospital Epidemiology - CDC - Staphylococcus aureus resistant to vancomycin. MMWR Morb. Mortal. Wkly. Rep., 51, 565-567, 2002; CHANG, S.; SIEVERT, D.M.; HAGEMAN, J.C.; BOULTON, M.L.; TENOVER, F.C.; DOWNES, F.P.; SHAH, S.; RUDRIK, J.T.; PUPP, G.R.; BROWN, W.J.; CARDO, D. & FRIDKIN, S.K. Brief report: infection with vancomycin-resistant Staphylococcus aureus containing the vanA resistance gene. N. Engl. J. Med., 348, 1342-1347, 2003). In Brazil, isolation of strains presenting intermediate resistance to vancomycin was reported (MAMIZUKA EM, OLIVEIRA GA. Isolation of strains of Staphylococcus aureus with reduced sensitivity to vancomycin in a Brazilian hospital. Pharm Bras 6: 7-8, 2000).

It has been suggested that the spread of MRSA in the hospital environment occurs mainly through cross-transmission between patients infected or colonized and the attending health team (NOGUERAS, M., MARINSALTA, N., ROUSSELL, M. & NOTARIO, R. Importance of hand germ contamination in health-care workers as possible carriers of nosocomial infections. Rev. Inst. Med. Trop. S. Paulo. 43: 149-152. 2001, WEBER, S., HERWALDT, L.A., MCNUTT, L.A. An outbreak of Staphylococcus aureus in a pediatric cardiothoracic surgery unit. Infect. Control Hosp. Epidemiol. 23: 77-81, 2002), podendo ocorrer também através da contaminação de fômites de uso comum e superficies (SCOTT, E. & BLOOMFIELD, S.F. The survival and transfer of microbial contamination via cloths, hands and utensils. J. Appl. Bacteriol. 68: 271-8, 1990; RIBEIRO, I. CASTANHEIRA, R.R. Treatment and prevention of Staphylococcus aureus. Port Pneumol. 5: 395-409., 2003).

The main risk factors associated with the development of infections caused by MRSA include hospitalization or recent surgery, hospitalization for long period, dialysis and application of intravenous drugs (RIBEIRO, I. CASTANHEIRA, R. R. Treatment and prevention of Staphylococcus aureus. Port Pneumol. 5: 395-409, 2003). After contamination, the bacteria initially colonize sites of entry, such as the skin, wounds, or areas of interface between invasive medical devices and the skin, such as catheters or probes. Infection spreads from this initial site and is carried to the interior of the body, where a systemic infection may be established (WENZEL RP, PERL TM: The significance of nasal carriage of Staphylococcus aureus and the incidence of postoperative wound infection. J Hosp Infect 31: 13-24, 1995). The preventive control of contamination of these entry sites through the direct topic application of antimicrobial agents is an important strategy (see above Ribeiro and Castanheira, 2003).

Although considered to be essentially hospital pathogens, it has been observed with great concern that extremely virulent strains of MRSA have migrated to the community (called community-associated MRSA or CA-MRSA), causing severe infections in healthy individuals such as children of school age, athletes, military and prison inmates (LINDENMAYER, J.M., SCHOENFELD, S., O'GRADY, R. & CARNEY, J.K. Methicillin-resistant Staphylococcus aureus in a high school wrestling team and the surrounding community. Arch. Intern. Med., 158, 895-899, 1998; SAID-SALIM, B.; MATHEMA, B. & KREISWIRTH B.N. Community-acquired methicillin-resistant Staphylococcus aureus: an emerging pathogen. Infect. Control Hosp. Epidemiol., 24, 451-455, 2003; ELLIS, M.W., HOSPENTHAL, D.R., DOOLEY, D.P., GRAY, P.J., MURRAY, C.K., 2004. Clin. Infect. Dis. Natural history of community-acquired methicillin-resistant Staphylococcus aureus colonization and infection in soldiers.39: 971-979; KAZAKOVA, S.V.; HAGEMAN, J.C.; MATAVA, M.; SRINIVASAN, A.; PHELAN, L.; GARFINKEL, B.; BOO, T.; MCALLISTER, S.; ANDERSON, J.; JENSEN, B.; DODSON, D.; LONSWAY, D.; MCDOMGAL, L.K.; ARDUINO, M.; FRASER, V.J.; KILLGORE, G.; TENOVER, F.C.; CODY, S. & JERNIGAN, D.B. A clone of methicillin-resistant Staphylococcus aureus among professional football players. N. Engl. J. Med., 352, 468-475, 2005; ROMANO, R.; LU, D. & HOLTON, P. Outbreak of community-acquired methicillin-resistant Staphylococcus aureus skin infections among a collegiate football team. J. Athl. Train., 41, 141-145, 2006). It appears that the transmission, in these cases, occurs mainly by physical contact, through minor skin lesion or abrasions. Such strains have been associated with skin infections and in soft tissues, such as cellulitis and abscesses (HEROLD, B.C.; IMMERGLUCK, L.C.; MARANAN, M.C.; LAUDERDALE, D.S.; GASKIN, R.E.; BOYLE-VAVRA, S.; LEITCH, C.D. & DAUM, R.S. Community acquired methicillin-resistant Staphylococcus aureus in children with no identified predisposing risk. JAMA, 279, 593-598, 1098). No entanto, têm sido descritos casos envolvendo bacteremias e pneumonias letais fulminantes em pacientes jovens e sadios (BOUSSAUD, V.; PARROT, A.; MAYAUD, C.; WISLEZ, M.; ANTOINE, M.; PICARD, C.; DELISLE, F.; ETIENNE J. & CADRANEL J. Life-threatening hemoptysis in adults with community-acquired pneumonia due to Panton-Valentine leukocidin-secreting Staphylococcus aureus. Intensive Care Med., 29, 1840-1843, 2003; KLEIN, J.L.; PETROVIC, Z.; TREACHER, D. & EDGEWORTH, J. Severe community-acquired pneumonia caused by Panton-Valentine leukocidin-positive Staphylococcus aureus: first reported case in the United Kingdom. Intensive Care Med., 29, 1399, 2003; VAN DER FLIER, M.; VAN DIJK, N.B.; FLUIT, A.C.; FLEER, A.; WOLFS, T.F. & VAN GESTEL J.P. Fatal pneumonia in an adolescent due to community-acquired methicillin-resistant Staphylococcus aureus positive for Panton-Valentine-leukocidin. Ned. Tijdschr. Geneeskd., 147, 1076-1079, 2003).

### On the phytotherapic treatment of infections.

Currently, the only commercially available pharmacological alternative for the treatment of complicated infections caused by multidrug-resistant bacteria are glycopeptides such as vancomycin and teicoplanin, which require intravenous administration and are considered to be ototoxic and nephrotoxic, besides being expensive.

Several studies have compared the efficacy of topic antibiotic activity of plant extracts and that of commercially available antimicrobial products recommended in clinical practice for the treatment of infections caused by MRSA. For example, Caelli and collaborators (2000) observed that, when considered as two distinct groups of patients initially infected by MRSA, one being subjected to treatment with plant extracts of Malaleuca species (nasal application at 4% and bath at 5%) and another group treated with traditional topic antimicrobials such as mupirocin (at 2%) and triclosan (control), there was a higher percentage of elimination of MRSA in patients treated with plant extracts. In an extensive investigative work, Quave and collaborators (2008) assessed 104 species of medicinal and non-medicinal plants from the south of Italy, and observed that the majority of those that showed anti-MRSA activity were identified as non-medicinal, i.e. not used for this purpose in traditional medicine. The values of the minimum inhibitory concentration (MIC) of plant extracts obtained from these species, however, varied from 256 to 512 µg/mL. In the same study, when the activity of these extracts was evaluated for their effectiveness against MRSA biofilms, it was observed that 10 species showed MIC values smaller than 32 µg/mL.

The antimicrobial activity of a methanol extract a plant species common in South Africa (*Helichrysum pedunculatum*) against several pathogenic bacterial species was evaluated. The sample presented a MIC value of 5000 µg/mL against a strain of MRSA (Aiyegoro O.A., Okoh A.I. Phytochemical Screening and Polyphenolic Antioxidant Activity of Aqueous Crude Leaf Extract of Helichrysum pedunculatum. International Journal of Molecular Sciences 2009; 10(11):4990-5001). The same study evaluated the possible synergic use of the same plant extract as adjuvant to the use of antibiotics commonly employed in the treatment of bacterial infections in clinical practice. No such synergism was observed; there was no change in normal values of MIC against MRSA for the antimicrobials tested.

### OBJECTIVES OF THE INVENTION

It is an objective of the present invention to provide plant extracts of *Kielmeyera aureovinosa* possessing antibiotic activity.

In particular, it is an objective of the present invention to provide plant extracts obtained from the roots of Kielmeyera aureovinosa possessing antibiotic activity.

Specifically, the plant extracts of *Kielmeyera aureovinosa* possessing antibiotic activity are obtained by extraction with organic solvent.

More specifically, the plant extracts of *Kielmeyera aureovinosa* possessing antibiotic activity are extracted with a solvent selected from the group including, but not limited to methanol, ethanol, ethyl acetate and hexane. Particularly, the plant extracts of *Kielmeyera aureovinosa* exhibit a minimum inhibitory concentration (MIC) against Staphylococcus aureus of less than or equal to 1 µg/mL tested by broth microdilution.

Specifically, the plant extracts of *Kielmeyera aureovinosa* of this invention are characterized by a concentration greater than 60% of chemical markers mammeisine and isomammeisine.

It is also an objective of the present invention to provide the compound 5-hydroxy-8,8-dimethyl-6- (iso-propyl)-4-aryl-4H-cyclohexen-(2,3-h]chromen-2-one denominated amarilin and is isolated from plant extracts of *Kielmeyera aureovinosa.*

Particularly, the minimum inhibitory concentration (MIC) of the test compound amarilin in broth microdilution against *Staphylococcus aureus* is less than or equal to 1 µg/mL.

It is another objective of the present invention to supply phytotherapic compositions comprising plant extracts of *Kielmeyera aureovinosa* and pharmaceutically acceptable excipients.

Particularly, the phytotherapic compositions of Kielmeyera aureovinosa of this invention are intended for topical administration.

More particularly, the pharmaceutical form of the phytotherapic compositions comprising plant extracts of *Kielmeyera aureovinosa intended* for topical use can be selected from the group including, but not limited to emulsion, gel-cream, ointment, cream, gel, soap and paste.

Particularly, the phytotherapic compositions comprising extracts of *Kielmeyera aureovinosa* of this invention are also intended for oral administration.

More particularly, the pharmaceutical form of phytotherapics comprising plant extracts of *Kielmeyera aureovinosa* for oral use, can be selected from the group including, but not limited to pill, capsule, syrup, suspension solution and tablet.

Another objective of the invention is the provision of pharmaceutical or veterinary compositions comprising the compound 5-hydroxy-8,8-dimethyl-6- (iso-propyl)-4-aryl-4H-cyclohexen-(2,3-h]chromen-2-one (amarilin) isolated and pharmaceutically acceptable excipients.

Particularly, the pharmaceutical or veterinary compositions comprising the compound amarilin may be intended for topical administration.

More particularly, pharmaceutical form of pharmaceutical or veterinary compositions comprising the compound amarilin for topical use , can be selected from the group including, but not limited to ointment, cream, gel, soap, lotion, emulsion, gel-cream and paste.

Particularly, the pharmaceutical or veterinary compositions comprising the compound amarilin may be intended for oral administration.

More particularly, the pharmaceutical form of pharmaceutical or veterinary compositions comprising the compound amarilin for oral administration can be selected from the group including, but not limited to powder, granulates, compressed tablets, capsule, solution, syrup and suspension.

Particularly, the pharmaceutical or veterinary compositions comprising the compound amarilin may be intended for parenteral administration.

More particularly, it is an objective of the present invention provide compositions as those described above for use in the prevention or treatment of bacterial infections.

It is a further objective of the present invention the use of plant extracts of *Kielmeyera aureovinosa,* the compound amarilin or of a composition of both to prepare a drug for prevention or therapy of bacterial infections.

Another objective of the present invention consists in a method for the prevention or treatment of bacterial infections with the use of plant extracts of *Kielmeyera aureovinosa,* the composed amarilin or of a composition I understand the same to prepare a drug for the prevention or treatment of bacterial infections.

### DESCRIPTION OF FIGURES

The following figures are part of this report and are included here in order to illustrate certain aspects of the invention. The object of the present invention may be better understood with reference to one or more of these figures, in combination with the detailed description of the preferred modality presented here.

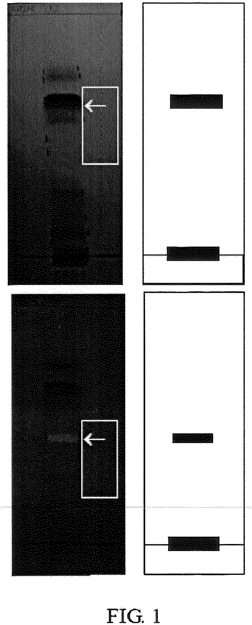

Figure 1 shows photographs of thin layer chromatography (TLC) of the ethanol extract of roots of *Kielmeyera aureovinosa* under ultraviolet light at wavelengths λ 254 nm and 365 nm, respectively. The eluent used was a mixture of toluene and ethyl acetate at a ratio of 93:7, where we the presence of the majority substance (at 254 and 365 nm) and coumarins (bands in blue at wave length of 365 - flagged with arrow ←). The diagrams in black and white highlight the bands to which attention is drawn.

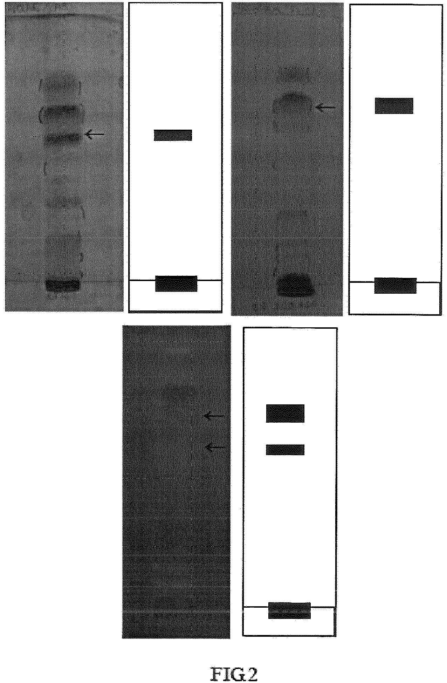

Figure 2 presents a photograph of three TLC plates evidencing the presence of terpenoids (purplish bands), flavonoids (yellow bands) and phenols (brown bands) as revealed with vanillin sulfuric, NPPEG and ferric chloride reagents respectively (evidenced arrow ←). The eluent used was a mixture of toluene and ethyl acetate at a ratio of 93:7. The diagrams in black and white highlight the bands to which attention is drawn.

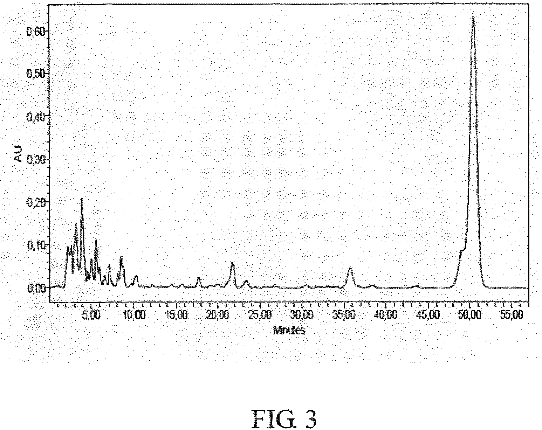

Figure 3 illustrates the chromatographic profile of the extract of *Kielmeyera aureovinosa* through the technique of high-performance liquid chromatography (HPLC). The profile presents a majority peak with retention time at 50 minutes, considered to be the chemical marker of the extract.

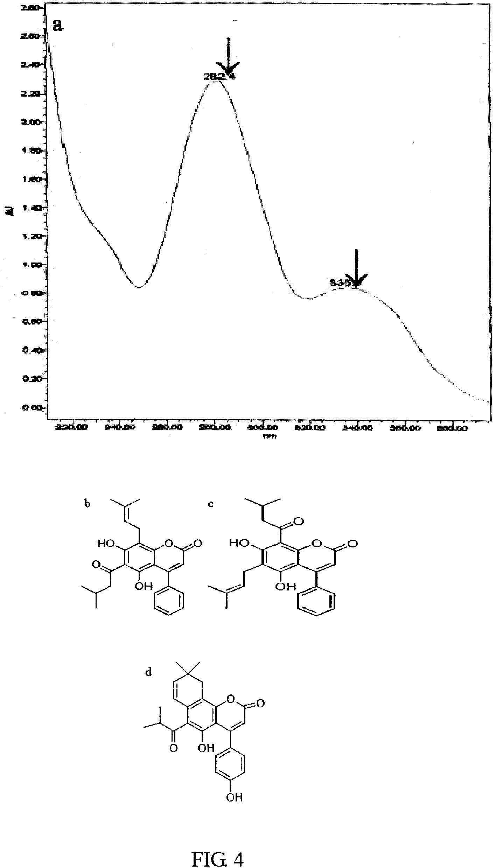

Figure 4a shows absorption maxima in an ultraviolet absorption spectrum of the chemical marker of the extract, showing maximum values of absorption in two wavelengths (282 and 335 nm λmax - flagged with ↓). The structure of substances isolated as chemical markers can also be found in this picture in (b) and (c) mammeisine and isomammeisine respectively. In (d) is shown the chemical structure of the novel compound 5-hydroxy-8,8-dimethyl-6-(iso-propyl)-4-aryl-4H-cyclohexen-(2,3-h]chromen-2-one, denominated amarilin, which was isolated from the main marker peak of the phytotherapic extract.

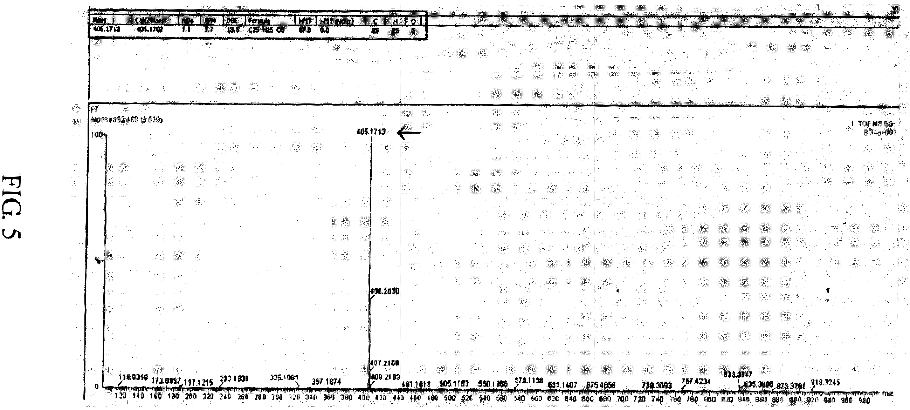

Figure 5 illustrates the mass spectrum of the isolated chemical marker of the ethanol extract of Kielmeyera sp., which presented, in negative mode, a molecular ion peak at m/z = 405.1713 (M-H) related to the molecular formula C₂₅H₂₆O₅ (evidenced by ↓).

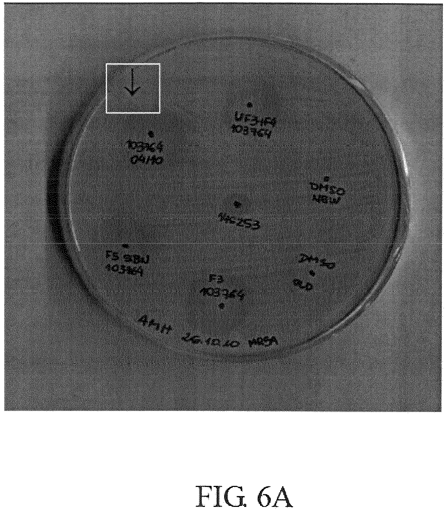

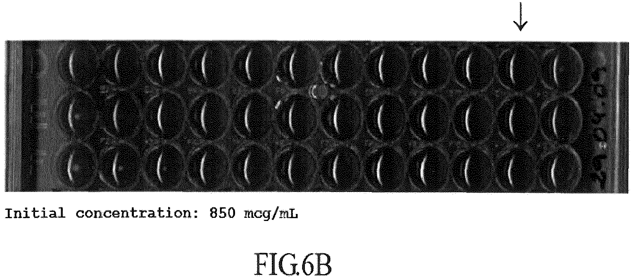

Figure 6 illustrates tests for antimicrobial activity. Figure 6A shows a Petri dish with culture medium where antimicrobial activity was evaluated qualitatively for different samples active by the methodology of diffusion in inoculated nutrient agar (Drop-Test). Pure DMSO samples are shown as zero control. Figure 6B shows in top view a sector of a 96-well plate used determine minimum inhibitory concentration (MIC) of extract EXT 103764 when tested against multidrug-resistant strains of MRSA (standard ATCC 33591). Initial Concentration: 850 µg/mL. Result obtained for MIC: 0.83 µg/mL (see arrow).

### DETAILED DESCRIPTION OF THE INVENTION

The plant from which the extract of the present invention was obtained, termed arbitrarily EXT 103764 or aureociclin, is a hitherto undescribed species of the genus Kielmeyera. It was harvested under the Special Authorization no. 16/2006 awarded to EXTRACTA Moléculas Naturais S/A by CGEN/MMA (Conselho de Gestão do Patrimônio Genético, Ministry of the Enviroment) and renewed by publication in the Brazilian Official Journal (Diário Oficial da Uniao, D.O.U. 15:55, January 22, 2009). In accordance with Brazilian Legislation, subsamples of the plant can be found under number 25756 at herbarium RFA at Federal University of Rio de Janeiro, which has been designated as faithful depositary by CGEN/MMA.

### Botanical Description of the plant Kielmeyera aureovinosa.

Plant *Kielmeyera aureovinosa,* Family *Clusiaceae, are* is a monoicous plant possessing glabrous aerial vegetative structures scarce in latex, which is thick and yellow. Plants of this species have a sparsely fasciculated underground root system, robust, papyraceous surface lamina, yellowish and defoliant. The plant is a shrub or small tree measuring 6 to 8 meters in height, diameter at breast level measuring 12 to 20cm, stem and lateral branches developed with a vinous metallic satin sheen with transverse defoliating golden bands in spiral, papyraceous membranes Young branches are thin, cylindrical to triangular-rounded and striated. The leaves are entire, alternal and spiral; petiole is slender, splined, with canalicules, with 2 to 4 cm in length; blade obovate to elliptical basis, with acute or cuneate, sometimes slightly asymmetric, apex rounded, obtuse sometimes apiculated, rarely acute or emarginated; thin midribs inprinted on the adaxial and abaxial surfaces; between 15 and 22 secondary veins on both sides, prominent on both surfaces; intersecondary stems and reticulation evident on both faces; orangey marginal rib (*in sicco*); translucent yellow (*in sicco*) segments and ovoid, obloides or fusiform, sparsely distributed in the vicinity of the margin. Inflorescences are present in non-umbelliform thyrse corimbiform pauciflorous, hemispheric; branches; pedicels articulated, striated with 1 to 1.5 cm in length. Flowers pentâmeric, general; floral buds ovoid, chalice dialissepalous, spawn, apex rounded, acute-ciliated, unequal, with dimensions of 0.35x0,55 -0,45x0.6 -0.5x0.7cm; corolla dialipetala, petals oblong-obovadas, apex rounded, truncated-ciliated, slightly asymmetrical, with dimensions of represents 1.4-2x1 -1.5cm; androecium polistemonous, thin fillets, pills, glabrous, anthers basifixed, are rimoses, obloides or obovoides, base and apex truncated with 0.1 to 0.15 cm length; gynoecium 3-carpel, glabrous ovary, semilunar hiatus, striatum, 3-locular, many ova in each locule, ca. 0.5 cm length, terminal stipules ca. 0.6 cm length, ovoid stigmatic papillae, ca. 0.1 cm in length. Fruits are not observed.

### Plant Extracts from Plants of Family Clusiaceae

Plants of the Family *CZusiaceae* are used in folk medicine for the treatment of abdominal disorders, back pain, colds, conjunctivitis, cough, dysmenorrhea, headache, snakebite and scorpion sting.

Chemical analysis of extract 103764 (Aureociclin), obtained from species *Kielmeyera aureovinosa,* Family *Clusiaceae* demonstrated the presence of coumarins, terpenoids, fatty acids and flavonoids. The chemical profile developed by CCF indicates the presence of flavonoids in the ethanol extract, as highlighted by yellow staining revealed through the reagent NPPEG (composed of two reagents NP "Naturstoff Reagenz A", ester 2-amino-acid ethyl difenilboric; and PEG (polyethylene glycol 4080) (Figure 1). Other groups of chemical substances were identified by CCF through their reactions with specific reagents (KOH 10% in ethanol and ferric chloride), staining indicated the presence of coumarins (bluish spot) and phenols (brown) (Figure 2).

The nature of chemical markers indicating the presence of neoflavonoids was characterized by absorption maxima at 282 and 335 nm λmax observed in the UV spectrum of the same (Figure 4a). The chemical marker showed retention time of 50 minutes. Its molecular weight was determined by the high resolution mass spectrometry as being 406, corresponding to the molecular formula C₂₅H₂₆O₅ as the presence of the molecular ion in negative mode m/z = 405.1713 (M-H) (Figure 5). The identification of a mixture of structures of two neoflavonoids or 4-aryl-coumarins, mammeisine isomammeisine (1) and (2) (Figures 4b and c, respectively), as being the majoritarian substances was confirmed by spectroscopic analyzes of Nuclear Magnetic Resonance.

Chemical fractionation allowed for evidencing of the natural antimicrobial activity of some fractions of the extract, which showed very significant low values for minimum inhibitory concentration (MIC). Through the methodology of broth microdilution, the a alcoholic extract (re-suspended in DMSO) of a sample of *Kielmeyera sp.* (Aureociclin), the plant extract of this patent application, presented value of MIC for antimicrobial activity of 0.83 µg/mL. Such activity for a crude plant extract is, up to the present, unprecedented. As mentioned above , currently mupirocin is widely used within the hospital environment as one of the main topical antistaphylococcal agents, presenting MIC value estimated at 0.5 µg/mL (Farias, W. V. , SADER, H. S. , RUDDER, I. L., PIGNATARI, A. C. Rev. Assoc. Med. Bras., Sensitivity pattern of 117 clinical isolates of Staphylococcus aureus from 12 hospitals. 43: 971-979, 1997). Yet that is the value assigned to the pure molecule of mupirocin, the synthesis of which is dependent on relatively expensive processes of extraction and purification. In addition, certain characteristics arising from the formulation of products containing mupirocin are highly restrictive to clinical use, such as the high nephrotoxicity of excipients used and impossibility of use in areas of burns.

The phytochemical study of the ethanol extract of the bioactive extract of this invention shows a composition rich in phenols, terpenoids and coumarins (mostly). Its MIC is less than 1 µg/mL for S. aureus-MRSA through the quantitative test of broth microdilution, while that of an antimicrobial agent (commercial hydrochloride ciprofloxacin) used as a reference presents MIC values of 1 µg/mL. The observation in a vegetal extract of antibiotic activity so intense against *S. aureus* is unprecedented until the moment.

In the present invention, other forms of preparation of the plant extract were also presented, since characteristics of polarity may modify the quality and quantity of extraction of substances and consequently, the activity.

The plant extract of the present invention demonstrated low cytotoxic activity in vitro in cultures of eukaryotic cells of mammals (cells of hamster ovary - CHO). With a concentration of extract of 12 µg/mL levels of cellular protection of 73%, on average, were obtained. Independent cytotoxicity Tests conducted in vitro on human fibroblasts and on murine fibroblasts (strain 3T3) showed cytotoxic concentrations of the order of 11 µg/mL for a protection of 80%.

The minimum inhibitory concentration presented by the plant extract of the present invention is in an order of magnitude below these cytotoxic concentrations. The high therapeutic index expected makes the plant extract of the present invention capable of use for the development of commercial, phytotherapeutic products already that cytotoxicity is an important limiting factor in the development of antimicrobial agents.

Despite some advances achieved in terms of active extracts against MRSA, the present invention proposes to contribute to the continuous search for more potent products against pathogenic micro-organisms.

### Description of the plant extract (aureociclin)

In a first method, the present invention relates to a plant extract obtained from *Kielmeyera aureovinosa* which possesses antibiotic activity. This extract was given the name of aureociclin.

Particularly, the plant extract can be obtained from any part of the plant *Kielmeyera aureovinosa,* by any method of extraction with organic solvent.

Preferably, the plant extract of the present invention is obtained the plant roots of *Kielmeyera aureovinosa.*

Methods that can be used to obtain the plant extract of *Kielmeyera aureovinosa* possessing antibiotic activity include, but are not limited to methods to cold extraction, as turbolization, maceration and percolation, either simple or fractionated. Optionally, methods may be used that employ hot extraction in open systems, such as infusion, turbolization and decoction, but not limited to these. It is also possible to obtain the plant extract of this invention by methods of hot extraction in closed systems, such as extraction under reflux and solvent extraction using heating.

Preferably, the extraction method used in the present invention consists in a combination of two or more methods mentioned above. More preferably, the extraction method used in the present invention comprises maceration and cold solvent extraction. For example, the extract may be prepared from the dry powder of roots placed in a maceration extractor to which is added a volume of solvent sufficient for homogenization of the mixture and complete immersion of powder in the solvent. The mixture is maintained in the extractor for a period of 10 to 50 hours, being subsequently filtered and concentrated in a rotatory evaporator at low pressure.

Particularly, the plant extract may be obtained by any of the methods mentioned above using an organic solvent selected from the group including, but not limited to oxygenated solvents, hydrocarbon solvents and halogenated solvents or aromatic.

In accordance with the present invention, oxygenated solvents may be products such as ketones, ethers, glycol and alcohol, hydrocarbon solvents may be aromatic hydrocarbons and aliphatic, including, but not limited to, for example, hexane, cyclohexane, toluene, benzene and derivatives thereof. In its turn, halogenated solvents may be derived from any hydrocarbon solvent which went through a process of halogenation, including, but not limited to, aril halide, alkyl chloride halide and cicloalquil halide.

Particularly, the plant extract of the present invention presents minimum inhibitory concentration (MIC) in test of broth microdilution against methicillin resistant *Staphylococcus aureus* of less or equal to 1 µg/mL.

More particularly, the plant extract of the present invention may be chemically characterized by the presence of compounds mammeisine and isomammeisine. Preferably, the concentration of the chemical markers mammeisine and isomammeisine is superior to 60 % in plant extracts of the present invention.

### Description of compound amarilin

In another form, the present invention refers to a chemical compound isolated from Aureociclin denominated amarilin. Particularly, the compound of the present invention has a molecular structure as the Formula I below: 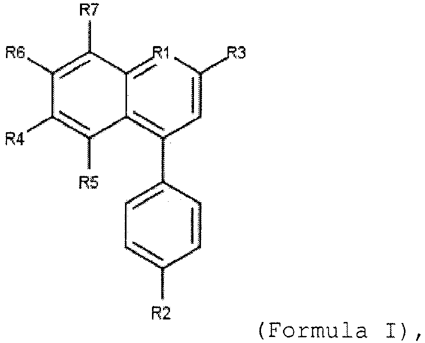 in which:
R1 is selected from the group comprising carbon or a heteroatom selected from the group comprising oxygen, nitrogen and sulfur.
R2, R3, R4, R5, R6 e R7 are independently selected from the group comprising hydrogen; cyclic or open alkyl, aromatic or alyphatic optionally substituted, cyclic or open alkynil, aromatic or aliphatic optionally substituted, aryl optionally substituted, cyclic or open halo alkyl optionally substituted, oxygen, sulphur, amine optionally substituted, hydroxyl, alkoxy cyclic or aliphatic, ariloxy optionally substituted, nitro, ciano, sulphonyl optionally substituted, carboxylic acid and amide. R6 and R7 may form an aliphatic or aromatic cycle optionally substituted.

More particularly, the compound of the present invention is the 5-hydroxy-8,8-dimethyl-6-(iso-propyl)-4-aryl-4H-cyclohexen-(2,3-h]chromen-2-one (amarilin), of formula I:

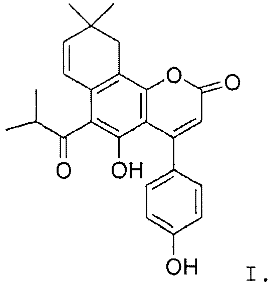

Particularly, the chemical compound amarilin is isolated from the extract aureociclin and, like it, has antibiotic activity. More particularly, the antibiotic activity of the compound amarilin presents minimum inhibitory concentration (MIC) in tests of broth microdilution against *Staphylococcus aureus* less or equal to 1 µg/mL.

### Description of the compositions

In another form, the present invention relates to compositions comprising phytotherapic extracts of *Kielmeyera aureovinosa* and pharmaceutically acceptable excipients.

As used in this invention, the employment of the term "pharmaceutically acceptable" means a non-toxic, inert solid, liquid or semi-solid excipient, diluent auxiliary formulation of any type, or simply an aqueous medium, such as sterile saline solution. Some examples of materials that can serve as pharmaceutically acceptable vehicles are sugars, such as lactose, glucose and sucrose, starch, such as corn starch and potato starch, cellulose and its derivatives, such as sodium carboxymethyl cellulose, ethyl cellulose and cellulose acetate, cyclodextrin; oils, such as peanut oil, cotton seed oil, sunflower oil, sesame oil, olive oil, corn oil and soybean seed oil; glycols, such as propylene glycol, polioils such as glicerineglicol, sorbitol, mannitol and polyethylene; esters, such as ethyl laurate, oleate; agar; adjuvant agents such as aluminum hydroxide and magnesium hydroxide; alginic acid; water free of pyrogen; isotonic saline, lactate Ringer's solution; buffer solutions of ethyl alcohol and phosphate, as well as other non-toxic substances used in pharmaceutical formulations.

Particularly, the compositions comprising phytotherapic extracts of *Kielmeyera aureovinosa* of this invention are intended for topical administration.

More particularly, the pharmaceutical form of phytotherapic compositions comprising plant extracts of *Kielmeyera aureovinosa* for topical use, can be selected from the group including, but not limited to emulsion, gel-cream, ointment, cream, gel, soap and paste.
Particularly, the compositions comprising phytotherapic extracts of *Kielmeyera aureovinosa* of this invention are intended for oral administration.

More particularly, the pharmaceutical form of phytotherapic compositions comprising plant extracts of *Kielmeyera aureovinosa* for oral use, can be selected from the group including, but not limited to powder, granulates, compressed tablets, capsule, solution, syrup and suspension.

In another form of the invention, pharmaceutical compositions are provided for human or veterinary use comprising the compound 5-hydroxy-8,8-dimethyl-6- (iso-propyl)-4-aryl-4H-cyclohexen-(2,3-h]chromen-2-one (amarilin) isolated and pharmaceutically acceptable excipients.

Particularly, the pharmaceutical or veterinary compositions comprising the compound amarilin may be intended for topical administration.

More particularly, pharmaceutical form of pharmaceutical or veterinary compositions comprising the compound amarilin for topical use, can be selected from the group including, but not limited to ointment, cream, gel, soap, lotion, emulsion, gel-cream and paste.

Particularly, the pharmaceutical or veterinary compositions comprising the compound amarilin may be intended for oral administration.

More particularly, pharmaceutical form of pharmaceutical or veterinary compositions comprising the compound amarilin for topical use, can be selected from the group including, but not limited to powder, granulate, pill, coated pill, capsule, solution, syrup and suspension.

Particularly, the pharmaceutical or veterinary compositions comprising the compound amarilin may be intended for parenteral administration.

Specifically, the compositions of the present invention may include any type of excipient used in the production of medicines in any one of pharmaceutical forms mentioned above, such as absorbent, diluents, binders, lubricants, disintegrants, sliding, plasticizers, coating agents, matrices for controlled release, solvents and co-solvents, wetting agents, emulsifying agents, surfactant agents, consistency donor, agents of tonicity, wetting agent, agents for expulsion of air, alkalinizing or acidifying agents, preservatives, antioxidants, bactericides, bacteriostatic, chelating agents, colorings and sweeteners.

Absorbents suitable for the compositions of this invention may be any substance added to absorb water present in extracts or to fix certain volatile compounds, such as essences. Examples are not exhaustive or limiting of such excipients are calcium phosphate, kaolin, magnesium carbonate, bentonite clay and talc.

The compositions of the present invention can understand, such as thinner, any product inert added to the formula to allow the obtaining of tablets or capsule filling with appropriate volumes and provide flow properties and compression necessary for the production, for example, but not limited to lactose, calcium phosphate (tribasic), starch, mannitol, calcium sulphate, Microcel^{®}, microcrystalline cellulose (Avicel^{®}), calcium phosphate, dibasic (Encompress Ditab^{®}), magnesium oxide, magnesium carbonate, talc, kaolin, calcium carbonate, dextrose, fructose, lactose, aspartame, cellulose, maltose, mannitol, guar gum, sorbitol, starch and sucrose.

Agglutination substances suitable for the compositions of this invention may be agents used to promote adhesion of particles during the granulation and compression of solid pharmaceutical forms, can also be used in compositions of the present invention, for example, carbopol, povidone, xanthan gum, gum arabic, alginic acid, sugar compressible, CMC-Na, ethyl cellulose, gelatin, methylcellulose, povidone (PVP), starch, pre-gelatinized starch and glucose liquid in the form of a solution, dispersion, or dust.

Disaggregants or disintegrants suitable for the compositions of this invention may be any component used to accelerate the disintegration and/or dissolution of the pharmaceutical form in biological fluids, for example, alginic acid, starch, sodium alginate, CMC-In, microcrystalline cellulose, croscarmelose sodium (Ac-Di-Sol^{®}), nedocromil sodium^{®} sodium glycolate Not more starch (Explotab^{®}) and crospovidona (Kollidon CL^{®}).

Lubricants suitable for the compositions of the present invention may be, for example, magnesium stearate, calcium stearate, stearic acid, talc and hydrogenated vegetable oil (ex. Lubritab).^{®}

Sliding substances suitable for the compositions of the present invention may be, for example, colloidal silica (Aerosil 200^{®}) and talc.

Plasticizers suitable for the compositions of this invention can be used together with polymers, to modify the temperature of phase transition and to facilitate the coalescence of films formed on granules, tablets or pellets. Non limitative examples of such agents are glycerin, trietilcitrate, dibutilftalate, silicone and PPG.

Coating Agents employed to coat compositions of this invention in the form of tablets, capsules, granules or pellets may be, for example, acetophtalate cellulose acetophtalate, ethyl cellulose, gellan gum, maltodextrine, methacrylates, methylcellulose, microcrystalline cellulose, shellacs, carrageenan gum, waxes, gelatin, cellulose derivatives (methyl or ethyl cellulose, cellulose, hidroxipropilmetilcelulose acetophtalate, cellulose acetate), copolymers of acrylic and methacrylic acid esters (Eudragit^{®} L100-types, RS 30D, RS PM, S100, among others), polyvinyl alcohol (PVA) and polyvinyl acetate.

Matrix forming agents for controlled release possibly employed in compositions of the present invention, with the purpose to obtain prolonged and/or controlled release of the active compound(s), can be HPMC, CMC-Na, xanthan gum, Carbopol^{®}, various types of Eudragit^{®}, agar-agar, derived polioxidoetilenics (PEO's), cyclodextrin, nanospheres and nanoparticles of any nature.

Solvents and co-solvents, such as alcohol, corn oil, cottonseed oil, glycerin, isopropyl alcohol, mineral oil, oleic acid, peanut oil, purified water and water for injection, among others, can also be used in compositions of the present invention.

Wetting agents suitable for the compositions of this invention may be any substance added with the purpose of reducing the surface tension in solid/liquid interface, for example, sodium lauryl sulphate (LSS), docusate sodium and polysorbates 20, 60, 80 (Tween^{®} 20, 60, 80).

Emulsifying Agents suitable for the compositions of the present invention may be, for example, glyceryl monostearate, cetyl alcohol and gelatin and auxiliaries, such as CMC-Na, MC, alginate and pectin.

Surfactant agents such as, for example, benzalkonium chloride, nonoxinol 10, octoxinol 9, polysorbate 80 and sodium lauryl sulphate are also suitable for the compositions of the present invention.

Consistency donor agents suitable for the compositions of this invention may be any substance used to increase the consistency of ointments, for example, cetyl alcohol, white wax, yellow wax, stearyl alcohol, paraffin wax, microcrystalline wax and cetilesther wax.

Tonicity Agents suitable for the compositions of this invention may be any substance used for obtaining solutions with osmotic characteristics similar to those of biological fluids to be administered by ocular, nasal, or parenteral routes, as NaCl (0.9 %), mannitol (5.07 %) and dextrose (5.51 %).

Humectant agents suitable for the compositions of this invention may be glycerin, propylene glycol and sorbitol.

Levigating agents suitable for the compositions of the present invention may be any liquid used as a facilitator agent in case of reduction of particles of the drug during the preparation of emulsions, oily bases, among others, for example, mineral oil (liquid petrolatum), glycerin, propylene glycol, PEG 400, cotton seed oil, castor oil and Polysorbate 80 (Tween ^{®} 80).

Agents for expulsion of air suitable for the compositions of this invention may be employed to expel the air in hermetically sealed containers or fluid formulations, to increase the stability, for example nitrogen (N2) and carbon dioxide (CO2).

Alkalizing or acidifying Agents, such as citric acid, ammonia solution, acetic acid, ammonium carbonate, fumaric acid, diethanolamine, hydrochloric acid (HCl), monoethanolamine, tartaric acid, potassium hydroxide (KOH), boric acid, sodium hydroxide (NaOH), sodium bicarbonate, sodium borate and tri-ethanolamine salts are also suitable for the compositions of the present invention.

Preservative agents that can be used in compositions of the present invention are, for example, antifungal agents, such as benzoic acid, sodium benzoate, methylparaben, butylparaben (when added), propylparaben (/ Nipasol), ethylparaben, sodium propionate, and antibacterials, such as benzalkonium chloride, benzethonium chloride, benzyl alcohol, cetylpyridinium chloride, chlorobutanol and phenol.^{®®}

Antioxidant Agents suitable for the compositions of the present invention can be selected from the group comprising butilhidroxianisol (BHA), butylhydroxytoluene (BHT), α-tocopherol, ascorbic acid, ascorbyl palmitate, sodium metabisulphite, ethylenediaminetetraacetic acid (EDTA), citric acid, cysteine, glutathione, vitamin C, sodium metabisulphite, cysteine and sodium thiosulphate.

The compositions of the present invention can understand still, as agents buffering agents, citrate buffer, phosphate and borate buffer. As colorings, flavorings and flavors can be used, for example, vanilla, menthol, oil of cinnamon, oil of anise and cocoa. Sweeteners may be, for example, aspartame, dextrose (glucose), mannitol, sorbitol, saccharin, cyclamates nedocromil sodium, sugar, acesulfame potassium, sucralose and stevioside.

The compositions of the present invention can comprise excipients such as bactericides, bacteriostatic, antioxidants, preservatives, buffers, stabilizers, pH adjusters, adjusters of osmolarity, anti-foaming agents and surfactants, as well as residues of inactivation or fractionation of antigens, components of growth media and solvents commonly used in the production of medicines. Examples of these types of components can be found in the book "The Handbook of Pharmaceutical Excipients", (RAYMOND C. Rowe, Publisher The Pharmaceutical Press).

A variety of routes of administration of the compositions described in this invention is available. The particular mode selected will depend on the active compound in particular selected, the dosage required for therapeutic efficacy and patient to whom the composition will be administered.

Particularly, the phytotherapic pharmaceutical and veterinary compositions described above are provided for use in the prevention or treatment of bacterial infections.

### Use of the Aureociclin extract and of the compound amarilin.

Considering the properties of the plant extract of *Kielmeyera aureovinosa* and of the compound amarilin isolated from the same, another aspect of the present invention is the use of compositions comprising the extract or isolated compound for the prevention or treatment of bacterial infections in animals.

Another aspect of the present invention is the use of the plant extract of *Kielmeyera aureovinosa* and of compound amarilin in the production vaccine-like medicines for prevention or treatment of infections, particularly bacterial infections.

### Methods for the prevention or treatment of bacterial infections.

It is yet another aspect of the present invention methods to prevent or treat bacterial infections in animals, more particularly in humans, using the Extract Aureociclin and compound amarilin, as well as compositions thereof.

The methods of the present invention, generally, can be practiced using any mode of administration biologically acceptable, as oral, rectal, sublingual, topical, nasal, transdermal, inhalation or any parenteral routes. The term "parenteral" includes subcutaneous, intravenous, intramuscular, epidural, irrigation pumps, release systems, or infusion. Particularly, topical, oral, parenteral, rectal, ocular and inhalation routes are preferred for administration of compositions of this invention.

### EXAMPLES

To allow a better understanding of the present invention and clearly demonstrate the technical advances obtained, the results of the various tests performed with respect to this invention are presented below as examples.

### Example 1: the Process of obtaining and preparing the extract of Kielmeyera aureovinosa.

The extract was prepared from 75 g of dry powder of roots, placed in an extractor vat where 750mL of ethanol 96°GL were added, assuring the homogenization of the mixture and complete immersion of the powder in the liquid. The immersion continued for 8h at room temperature, after which time the first change of solvents was performed. The change was repeated after another 16h completing a cycle of 24h. The extracts from both changes were concentrated in a rotary evaporator at low pressure at a temperature of 50°C to 70°C. Approximately 6g of dry extract were recovered (yield of 8%).

Purification of the extract was performed using column chromatography on silica gel in hexane. A quantity of 5.3g of extract was applied using a gradient of increasing polarity with ethyl acetate until a proportion of 15 % was reached.

### Physical Characteristics of extract:

The plant extract shows yellow color, is soluble in ethanol and has a pH between 6.0 and 7.0.

### Example 2: HPLC profile of extract

The HPLC chromatographic profile was obtained (Figure 3) for the extract containing the chemical marker identified as a mixture of neoflavonoids. This presents a retention time of 50 minutes under the conditions described in the list of figures.

### Example 3: UV Spectrum and structure of the neoflavonoids

The graph of absorption maxima in the Ultra Violet spectrum was obtained (Figure 4a). The neoflavonoids mammeisine (Figure 4b) and isomammeisine (Figure 4c) exhibit absorption maxima in ultraviolet, in wavelengths of 282 and 335 nm λmax, *as can* be seen in Figure 4a.

On the other hand, the novel substance amarilin (Figure 4d) showed, absorption maxima at 233.1 and 286.3 nm λmax (not shown in Figure 4).

### Example 4: Chromatographic Profile of the extract of Kielmeyera aureovinosa

The chromatographic profile of the extract of *Kielmeyera aureovinosa* was analyzed by high-performance liquid chromatography (HPLC). The method of analysis included an isocratic mobile phase (63% to 37% of acetonitrile: water acidified with phosphoric acid to 0.1%). The concentration of the sample in the mobile phase was 1.0 mg/mL. The column used was C18 ACE, with dimensions of 250mm X 4.6mm, 5 µm, the duration time of the run was 60 minutes. The majoritarian peak with retention time of 50 minutes was considered as a marker of the chemical extract (Figure 3).

### Example 5: Mass spectrum of the isolated chemical marker of the ethanol extract of Kielmeyera sp.

The mass spectrum of the isolated chemical marker of the ethanol extract of Kielmeyera *sp* showed, in negative mode, a molecular ion peak at m/z = 405.1713 (M-H) relative to the molecular formula C25H26O5 (Figure 5). The samples were analyzed by UPLC-EM in a Synapt in acquisition mode MSE, using electronspray ionization (ESI) in negative mode as a source of ionization. The conditions of the analysis included an Acquity BEH C18 column, with dimensions of 2.1x50 mm, 1.7 µm; mobile phase: A: H2O/0.1% Formic Acid; B: ACN/0.1% Formic Acid; gradient: 50-95% B in 5 minutes, column temperature: 40°C; injection volume: 7.5 µL (mode of injection Needle Overfill), with run time 5 min. The settings of the mass spectrometer were: capillary voltage: 3.5 kV, cone voltage: 35 V, desorption temperature: 350Â°C, desorption gas flow: 750 L/h; gas flow of the cone: 30 L/h; collision energy (Trap): 6.0 eV and collision energy (Transfer): 4.0 eV. The samples of extract EXT 103764, were dissolved in H2O/ACN 80:20 to approximately 2 mg/mL and subsequently diluted in water to 0.1 mg/mL. This was identified as a mixture of mammeisine and isomammeisine, two isomers, the structures of which are illustrated in Figures 4B and 4C, respectively.

### Example 6: Evaluation of anti-MRSA activity of the main extract

Quantitative evaluation of the antibacterial activity was performed by broth microdilution methodology for the determination of the minimum inhibitory concentration (MIC), according to the manual of the Clinical and Laboratory Standards Institute (CLSI, 2006). The ethanol extract was dissolved in DMSO at a concentration of 10mg/mL, from which serial dilutions were prepared (ratio 1:2) in 96-well sterile plates, geometry 12x8 wells. To each well were added 10 µL of a bacterial suspension containing approximately 7x10⁶ cells/mL, prepared from the recent growth of strain ATCC 33591 of MRSA in nutrient agar. After incubation in a bacteriological incubator at 36°C for 48 h, the plates were revealed by addition of 25 µL Resazurin (7-Hydroxy-3H-phenoxazin-3-one 10-oxide) to 0.01% in each well, followed by incubation at 36oC for 30 minutes. The direct visualization of the plate demonstrated that there was inhibition of bacterial growth in all concentrations down to 0.83 µg/mL, which is considered as the MIC of the crude extract of the tested strain of MRSA. The experiments were performed in quadruplicate, in different periods.

### Example 7: Bioautography

A TLC plate (silica gel) was previously prepared by elution of the ethanol extract of a sample of *Kielmeyera.* The solvent system used was toluene:ethyl acetate (93:7). Then, after drying the plate, 30.0 mL of suspension of fused Trypticase Soy Agar (TSA) containing approximately 105 cells /mL was dispensed. The plate was incubated at 36°C for 24 h. Then, 5.0 mL of dye Resazurin to 0.02% was applied over the entire length of the plate, followed by further incubation at 36°C for 30 minutes. The plate shows an area of inhibition of bacterial growth (bluish), corresponding to Rf = 0.54 cm.

### Example 8: Evaluation of antibiotic activity against the species M. fortuitum ATCC 6841, M. massiliense CRM0019 and CRM 0270, M. abscessus ATCC 1997 and M. smegmatis INCQS 061.

The strains used were standardized and derived from specimens of clinical significance, presenting high degree of multidrug resistance. Also included was a strain of *M. bovis* INCQS 062, since this species is recommended, according to the technical standards of the National Health Surveillance Agency (ANVISA), for the assessment of effectiveness of disinfectants for hospital use.

After a series of tests, which consisted of the direct assessment of crude extracts and fractions obtained by chemical fractionation of extracts, directed by antimicrobial evaluation tests, the following results were obtained:

**Table 1. MIC of the more active extracts and fractions observed against strains of M. tuberculosis and MCR. (Plants K.aureovinosa, K.a.; K.rizinniana, K.r.; Solanum flaccidum, S.f.)**

| **Microorganisms Tested** | **MIC Extracts/Fractions Tested (pg/ml)** | | | | | |
|---|---|---|---|---|---|---|
| | **F151-159 (103764) (K.a.)** | **EE118085 02/09 (K.r.)** | **EE145117 K.a.)** | **F55-72 +F6783 (103764) (K.a.)** | **F60-89 (103764) (K.a.)** | **EE106087 CF (S.f.)** |
| *Mycobacterium tuberculosis* H37Rv Strain ATCC 27294 | 25 | ≥100 | 50 | 25 | 6.25 | 6.25 |
| *Mycobacterium fortuitum* ATCC 6841 | 25 | ≥100 | 25 | 100 | 100 | 3.12 |
| *Mycobacterium abscessus* ATCC 1997 | 3.12 | ≥100 | 6.25 | 100 | 1.56 | 12.5 |
| *Mycobacterium massiliense* CRM 0019 | 3.12 | ≥100 | 12.5 | 100 | 1.56 | 12.5 |
| *Mycobacterium massiliense* CRM 0270 | 25 | ≥100 | 25 | 100 | 100 | N.A. |
| *Mycobacterium smegmatis* INCQS 061 | 3.12 | 100 | 6.25 | 100 | 3.12 | 3.12 |
| *Mycobacterium bovis* INCQS 062 | 0,39 | 1.56 | 0,39 | 0,39 | 0.78 | 12.5 |

Table I shows that extracts of at least two plant species have a very significant potential as anti-mycobacterial: *Kielmeyera aureovinosa* (Aureociclin) and Solanum flaccidum, another Brazilian Atlantic Rainforest plant in EXTRACTA Chemical Biodiversity Bank.

In relation to the assessment of species *Kielmeyera aureovinosa,* it was observed that the crude extract from the root of the plant (Aureociclin) showed substantial activity against *M. tuberculosis* and all strains of MCR evaluated, as well as for M. *bovis.* The fractions of this extract showed activities even more significant. For M. *tuberculosis,* the largest value of MIC found was 25 µg/mL; the lower value was 6.25 µg/mL. For the MCR, the observed values were even more expressive, varying from 25 µg/mL to 1.56 µg/mL.

### Example 9: Toxicological Tests of extract aureociclin

### a) Acute dermal irritation in rabbits for Aureociclin

The plan of studies was based on the Guidelines *OECD* 404 and the objective was to evaluate the harmful effects, reversible or not, arising from acute dermal exposure to Aureociclin in rabbits. The method consisted in topical application of the test substance, by the dermal route, a single time, to *Oryctolagus cuniculus* females, treated with a concentration of 80µg/mL of the test substance.

The test substance was applied, in the proportion of 0.5mL/area treated with syringe and spatula, appropriate for a quadrant of approximately 6 cm² of previously shaved dermis, in a group of three test-systems. The application site was preserved with dressing during 4 hours. An area equivalent to the application of the test substance was left intact in antimere opposite to serve as control.

All the test-systems were evaluated for the presence of signs of erythema, abrasion and edema at 60 minutes, 24 hours, 48 hours and 72 hours after removal of the dressing. After 24 hours, all test-systems presented mild erythema (barely noticeable) with the skin reddish in the whole area of application. Once detected a sign of irritation resulting from the application of the test substance, the test was extended by 14 days. It was observed that the redness subsided 10 days after administration of the test substance.

It was also detected mild desquamation of the treated skin, beginning about the fifth day and observed throughout the observation period. During the same period of exposure to the test substance and observation of test systems, no detected sign of edema in the treated area could be observed when compared to the control area.

The administration of acute dermal Aureociclin in the studied concentration of 80 µg/mL did not induce mortality, nor discomfort and/or pain were perceived during the test. At the end of the test, the test-systems were subjected to euthanasia by injectable anesthetics. In conclusion, the results obtained demonstrated that the acute dermal exposure to Aureociclin in a concentration of 80 µg/mL to rabbits, the New Zealand breed, female, did not cause death and does not induce systemic clinical alterations.

**Table 2: Occurrence of erythema in test-systems of the group treated with aureociclin.**

| **Time** | Right Antimere | | | Right Antimere | | |
|---|---|---|---|---|---|---|
| | Test Substance | | | Control | | |
| | Aureociclin | | | - | | |
| | Erythema Test | | | | | |
| | KA1/02 | KA1/04 | KA1/06 | KA1/02 | KA1/04 | KA1/06 |
| **1H** | 0 | 0 | 0 | 0 | 0 | 0 |
| **1 Day** | 1 | 1 | 1 | 0 | 0 | 0 |
| **2 Days** | 1 | 1 | 1 | 0 | 0 | 0 |
| **3 Days** | 1 | 1 | 1 | 0 | 0 | 0 |
| **4 Days** | 1 | 1 | 1 | 0 | 0 | 0 |
| **5 Days** | 1 | 1 | 1 | 0 | 0 | 0 |
| **6 Days** | 1 | 1 | 1 | 0 | 0 | 0 |
| **7 Days** | 1 | 1 | 1 | 0 | 0 | 0 |
| **8 Days** | 1 | 1 | 1 | 0 | 0 | 0 |
| **9 Days** | 1 | 1 | 1 | 0 | 0 | 0 |
| **10 Days** | 1 | 1 | 1 | 0 | 0 | 0 |
| **11 Days** | 0 | 0 | 0 | 0 | 0 | 0 |
| **12 Days** | 0 | 0 | 0 | 0 | 0 | 0 |
| **13 Days** | 0 | 0 | 0 | 0 | 0 | 0 |
| **14 Days** | 0 | 0 | 0 | 0 | 0 | 0 |

### B) Acute eye irritation for aureociclin in rabbits

The study was based on the Guidelines OECD 405 and aimed to assess the harmful effects, reversible or not, arising from acute eye exposure to Aureociclin in rabbits.

Three rabbits of the New Zealand breed, female, were treated with Aureociclin only once, at the concentration of 80 µg/mL. The method consisted in the application, with graduated syringe of the test substance into the conjunctive sac of the right eye, after the gentle lowering of the inferior eyelid. The collateral eye (left) was used as a negative control. At the end of a 24-hour exposure period, the residue of the test substance was removed carefully with the aid of gauze soaked in deionized water. Each test-system was evaluated separately for the presence of lesions on the cornea, iris, conjunctiva and eyelids, as well as other local and/or severe systemic changes.

The eyes were examined after 1, 24, 48 and 72 hours after administration of the test substance and daily during the remaining period of observation (6 days). To assist in the assessment of ocular reactions, fluorescein eye drops were used in the evaluations of 24, 48 and 72 hours. The evaluation of ocular reactions was based on the scale of Draize, which provides a breakdown in graduation of ocular reactions, such as: opacity, area of cornea involved, iritis, hyperemia, conjunctivitis and secretion.

The administration of ocular Aureociclin at 80 µg/mL did not induce mortality, nor change of clinical signs. The test substance presented index of ocular irritation of 0.0 on the scale of Draize, being considered, therefore, nonirritating when applied at a concentration of 80 µg/mL to the eye in rabbits.

In conclusion, the results obtained showed that acute eye exposure to Aureociclin in the concentration of 80 µg/mL to adult rabbits, New Zealand breed, female, did not induce irritation and/or corrosion during the observation period (6 days).

### c) Acute Toxicity for Aureociclin in rats.

The study aimed at evaluating the toxic effects resultant from exposure to acute dermal Aureociclin in rats. This test was based on the Guidelines OECD 402 and was conducted according to the plan of study 42011.

The method consisted in topical application of the test substance, by the dermal route, a single time, in a group of male test-systems evaluated only in higher concentrations (250 µg/mL). In parallel, a control group received only the placebo of test substance (Placebo Aureociclin). The application site was preserved with dressing for approximately 24 hours. Body weight and consumption of water and ration of test systems were recorded.

At the end of the observation period of 14 days, the test-systems were subjected to euthanasia and examined macroscopically. The acute administration of the test substance in the tested concentration was evaluated, as well as that to the placebo Aureociclin in test-systems of control and treated group. No changes were induced in clinical evaluations, on body weight and consumption of food and water capable of bearing a relationship to toxicity and no lethality was observed. In addition, macro- or microscopic alterations were not observed in relationship with exposure to the test substance. In conclusion, the results obtained showed that the administration of dermal Aureociclin to skin of *Rattus novergicus* in concentration of 250 µg/mL did not promote changes capable of relationship with toxicity.

**Table 3: Average body weight (g) in control group and group treated with Aureociclin.**

| Time | **Groups** | | | | | |
|---|---|---|---|---|---|---|
| | **Control** | | | **250 µg/mL** | | |
| | Average | S.D. | N | Average | S.D. | N |
| 0 | 281.30 | 20.63 | 5 | 282.02 | 14.24 | 5 |
| 1 | 287.54 | 20.37 | 5 | 301.14 | 15.76 | 5 |
| 2 | 325.28 | 26.88 | 5 | 326.00 | 15.63 | 5 |

**Table 4: Average weekly consumption of ration (g) per test-system of control groups and treated with Aureociclin.**

| TIME | **Groups** | | | | | |
|---|---|---|---|---|---|---|
| | **Control** | | | **250 µg/mL** | | |
| | Average | S.D. | N | Average | S.D. | N |
| Total | 150.40 | 18.66 | 2 | 171.72 | 3.74 | 2 |

**Table 5: Consumption of water (mL) average weekly per test-system of control groups and treated with Aureociclin.**

| TIME | **Groups** | | | | | |
|---|---|---|---|---|---|---|
| | **Control** | | | **250 µg/mL** | | |
| | **Average** | **S.D.** | **N** | **Average** | **S.D.** | **N** |
| Total | 272.60 | 8.64 | 2 | 247.22 | 6.69 | 2 |

**Table 6: Body Weight (g) of test-systems of control groups and treated with Aureociclin.**

| **Time** | **Control Group** | | | | |
|---|---|---|---|---|---|
| | **Whole** | **Head** | **Back** | **Tail** | **Dorsal** |
| 0 | 294.80 | 269.70 | 250.50 | 292.70 | 298.80 |
| 1 | 298.10 | 270.50 | 262.10 | 296.40 | 310.60 |
| 2 | 335.80 | 305.30 | 288.90 | 349.90 | 346.50 |

| **Group 250 µg/mL** | | | | | |
|---|---|---|---|---|---|
| **Time** | **Whole** | **Head** | **Back** | **Tail** | **Dorsal** |
| 0 | 298.20 | 295.50 | 272.60 | 278.00 | 265.80 |
| 1 | 318.60 | 312.10 | 284.60 | 284.60 | 305.80 |
| 2 | 339.80 | 334.90 | 301.10 | 320.60 | 333.60 |

### d) Cytotoxicity

Murine 3T3 Fibroblasts were seeded in two 96-well plates. One of these plates was used to provide the curve of cytotoxicity of control substance Duodecil Sodium sulphate (SDS), and the other provides the first profile of cytotoxicity of this cell lineage in the test concentrations used. This first profile provides the window of concentration (rangefinder) where the IC50 can be determined in a second moment (IC50 finder).

**Table 7: IC50 Finder Result**

| Dilution Factor: 1.78 | | | | | | | | |
|---|---|---|---|---|---|---|---|---|
| **(µg/ml)** | C1 | C2 | C3 | C4 | C5 | C6 | C7 | C8 |
| | 100.0 | 56.2 | 31.6 | 17.7 | 10.0 | 5.6 | 3.1 | 1.8 |
| **Relative Viability (%)** | -13,9 | -15,6 | 9.5 | 58.8 | 72.1 | 84.1 | 94.9 | 94.8 |

| | | | | | | | | |
|---|---|---|---|---|---|---|---|---|
| Indices of in vitro cytotoxicity : IC20 = 11.00 µg/ml; IC50 = 18.00 µg/ml; IC80 = 20.00 µg/ml LD20 = 257.87 mg/kg; LD50 = 309.71 mg/kg; LD80 = 322.09 mg/kg. | | | | | | | | |

The above results indicate that the test substance has very low toxic activity, being recommended care only from the concentration of 8 µg/mL, much above the minimum inhibitory concentration, which is less than 1 µg/mL, thus indicating a high therapeutic index.

### Example 10: Topical Compositions of Aureociclin

**Table 8: Cream Composition**

| **Component** | **Quantity** |
|---|---|
| **Oily Phase** | |
| Glyceryl monostearate | 5 g |
| Cetostearyl Alcohol | 10 g |
| Cetostearyl Alcohol Ethoxylate | 2.5 g |
| Solid Petroleum Jelly (vaseline) | 2 g |
| Ethyl hexyl stearate | 3 g |
| Phenoxyethanol/methylchloroisothiazolinone Phenoxyethanol + Isothiazolinone Compounds | 0.4 g |

| **Aqueous Phase** | |
|---|---|
| Propylene glycol | 5 g |
| Purified Water | qsp 100 g |

| **Phase C** | |
|---|---|
| Active Plant Extract (*) (from 8 to 250 µg/mL ) | qs |

| | |
|---|---|
| (*) (1mg/ml of standard extract comprises 60% in marker molecules) | |

**Table 9: Ointment composition**

| **Component** | **Quantity** |
|---|---|
| Anhydrous Lanolin | 30.0 g |
| Butylhydroxytoluene (BHT) | 0.05 g |
| Solid vaseline | Q.s.p. 100.0 g |

## Claims

1. Plant Extract **characterized in that it is** obtained from *Kielmeyera aureovinosa* and has antibiotic activity.

2. Plant Extract, according to claim 1, **characterized in that it is** obtained from the roots of *Kielmeyera aureovinosa.*

3. Plant Extract, according to claim 1 or 2, **characterized in that it is** an extract obtained by extraction with organic solvent.

4. Plant Extract, according to claim 3, **characterized in that the** solvent is an alcohol.

5. Plant Extract, according to any claim 1 to 3, **characterized in that the** solvent is selected from the group comprising, but not limited to methanol, ethanol, ethyl acetate and hexane.

6. Plant Extract, according to any one of claims 1 to 5, **characterized in that** its minimum inhibitory concentration (MIC) in a broth microdilution assay against *Staphylococcus aureus* is less than or equal to 1 µg/mL.

7. Plant Extract, according to any one of claims 1 to 6, **characterized in that** the concentration of chemical markers mammeisine and isomammeisine is greater than 60%.

8. Compound **characterized by** Formula I: 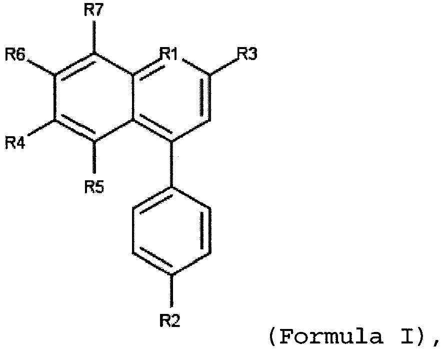 in which:
R1 is selected from the group comprising carbon or a heteroatom selected from the group comprising oxygen, nitrogen and sulfur.
R2, R3, R4, R5, R6 e R7 are independently selected from the group comprising hydrogen; cyclic or open aromatic or
alyphatic optionally substituted alkyl, cyclic or open aromatic or aliphatic optionally substituted alkynil, optionally substituted aryl, cyclic or open optionally substituted haloalkyl, oxygen, sulphur, optionally substituted amine, hydroxyl, cyclic or aliphatic alkoxy, optionally substituted ariloxy, nitro, ciano, optionally substituted sulphonyl, carboxylic acid and amide;
R6 and R7 may form an optionally substituted cyclic aliphatic or aromatic chain.

9. Compound, according to claim 8, **characterized in that it is** 5-hydroxy-8,8-dimethyl-6- (iso-propyl)-4-aryl-4H-cyclohexen-(2,3-h]chromen-2-one:

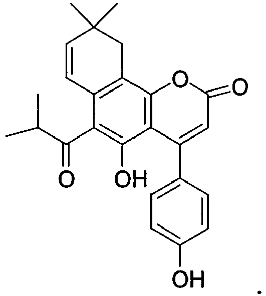

10. Compound, according to claim 8 or 9, **characterized by** having antibiotic activity.

11. Compound, according to claim 8 or 9, **characterized in that** its minimum inhibitory concentration (MIC) in broth microdilution assay against *Staphylococcus aureus* is less than or equal to 1µg/mL.

12. A phytotherapic composition **characterized in that** it comprises a plant extract as defined in any one of claims 1 to 7 and pharmaceutically and veterinarily acceptable excipients.

13. A composition, according to claim 12, **characterized in that** it is for topical administration.

14. A composition, according to claim 13 **characterized being** selected from the group including, but not limited to emulsion, gel-cream, ointment, cream, gel, soap and paste.

15. A composition, according to claim 12, **characterized in that** it is for oral administration.

16. A composition, according to claim 15, **characterized in that it is** selected from the group comprising, but not limited to pills, capsules, syrups, suspension solution and tablets.

17. A composition, in accordance with any one of claims 11 to 16, **characterized in that** it comprises a plant extract as defined in any one of claims 1 to 7 at a concentration of 8.0 µg/g to 2.5 mg/g.

18. A pharmaceutical or veterinary composition **characterized in that** it comprises the active compound as defined in any one of claims 8 to 11 and pharmaceuticaly and veterinarily acceptable excipients.

19. A composition, according to claim 18, **characterized in that** it is for topical administration.

20. A composition, according to claim 19, **characterized in that it is** selected from the group including, but not limited to emulsion, gel-cream, ointment, cream, gel, soap and paste.

21. A composition, according to claim 18, **characterized in that it is** intended for oral administration.

22. A composition, according to claim 21, **in that it is** selected from the group including, but not limited to powder, granulate, compressed tablet, pill, capsule, solution, syrup and suspension.

23. A composition, according to claim 18, **characterized in that it is** for parenteral administration.

24. A composition, according to any one of the claims 18 to 23, **characterized in that** it comprises a compound as defined in any one of claims 8 to 10 in concentration between 2.0 µg/g and 2.0 mg/g.

25. A composition, according to any one of claims 12 to 24, **characterized in that it is** for use in the prevention or treatment of bacterial infections.

26. Use of plant extracts as defined in any one of claims 1 to 7, or compounds as defined in any one of claims 8 to 11 or of a composition as defined in any one of claims 12 to 24 **characterized in that it is** for preparing a medicine for the prevention or treatment of bacterial infections.

27. Method for prevention or treatment of bacterial infections in animals **characterized in that** it comprises the administration of a pharmaceutical or veterinary effective amount of a plant extract as defined in any one of claims 1 to 7, of a compound as defined in any one of claims 8 to 11, or of a composition as defined in any one of claims 12 to 23.
